Europäisches Patentamt

European Patent Office

Office européen des brevets

(19)

(11) **EP 0 925 257 B1**

(12) **FASCICULE DE BREVET EUROPEEN**

(45) Date de publication et mention
de la délivrance du brevet:
**23.04.2003 Bulletin 2003/17**

(21) Numéro de dépôt: **97931874.8**

(22) Date de dépôt: **04.07.1997**

(51) Int Cl.$^7$: **C01B 33/193**, A61K 7/16

(86) Numéro de dépôt international:
**PCT/FR97/01207**

(87) Numéro de publication internationale:
**WO 98/001391 (15.01.1998 Gazette 1998/02)**

(54) **SILICE ABRASIVE UTILISABLE DANS LES DENTIFRICES**

IN ZAHNPASTEN VERWENDBARE ABRASIVE KIESELSÄURE

ABRASIVE SILICA TO BE USED IN TOOTHPASTE

(84) Etats contractants désignés:
**BE DE ES FI FR GB NL**
Etats d'extension désignés:
**RO SI**

(30) Priorité: **05.07.1996 FR 9608383**

(43) Date de publication de la demande:
**30.06.1999 Bulletin 1999/26**

(73) Titulaire: **RHODIA CHIMIE**
**92408 Courbevoie Cédex (FR)**

(72) Inventeurs:
• **AMICHE, Frédéric**
**F-92420 Vaucresson (FR)**

• **DROMARD, Adrien**
**F-69006 Lyon (FR)**
• **CHEVALLIER, Yvonick**
**F-69270 Fontaines Saint Martin (FR)**

(74) Mandataire: **Fabre, Madeleine-France**
**Rhodia Services,**
**Direction de la Propriété Industrielle,**
**40, rue de la Haie-Coq**
**93306 Aubervilliers Cedex (FR)**

(56) Documents cités:
**WO-A-95/18066**          **WO-A-96/09809**
**US-A- 5 225 177**

**Description**

La présente invention a pour objet un procédé de préparation d'une silice précipitée abrasive, utilisable notamment dans les compositions dentifrices, notamment pour usage fréquent.

Des silices d'abrasivité élevée sont usuellement mises en oeuvre dans les compositions dentifrices en raison du pouvoir nettoyant performant qu'elles confèrent auxdites compositions ; toutefois en raison des risques de dommages que peuvent créer de telles silices aux tissus oraux par de fréquents brossages, on est à la recherche de silices qui, tout en conservant un pouvoir nettoyant élevé en formulation, présentent une abrasivité intérieure à celle des silices standard pour dentifrices.

La demanderesse a trouvé un procédé pour préparer une silice permettant de résoudre ce problème.

Le procédé selon l'invention, vise la préparation d'une silice précipitée, utilisable comme agent abrasif dans les compositions dentifrices, silice présentant

- une surface spécifique BET de 60 à 150m$^2$/g, de préférence de 70 à 130m$^2$/g ;
- une abrasivité RDA de 30 à 90, de préférence de 40 à 80, tout particulièrement de 40 à 70 ;
- un indice de réfraction de 1,440 à 1,450 ;
- une prise d'huile DOP de 115 à 170 ml/100g, de préférence de plus de 120 ml/100g à 170 ml/100g ;
- un diamètre médian de particules $d_{50}$ de 6 à 13µm, tout particulièrement de 7 à 12µm ;
- et, lorsqu'elle est formulée à 10% en poids dans une composition dentifrice, un rapport pouvoir nettoyant / abrasivité RDA dans la composition dentifrice supérieur à 1,5 ;

par réaction d'un silicate de métal alcalin M , de rapport SiO$_2$ / M$_2$O de 2 à 4, de préférence de 3 à 3,8 , avec un agent acidifiant, éventuellement mûrissement de la bouillie de silice formée, séparation et séchage de la suspension de silice récupérée et broyage si nécessaire jusqu'à obtention d'un diamètre médian $d_{50}$ de particules de 6 à 13µm, de préférence de 7 à 12µm, l'opération de formation de la bouillie de silice étant réalisée selon les étapes successives suivantes :

- a) mise en oeuvre d'un pied de cuve initial constitué d'eau, d'un sel électrolyte et d'une fraction de la quantité totale de silicate engagée correspondant à un taux de consolidation TC

$$TC = \frac{\text{quantité totale de silicate engagée, exprimée en SiO2}}{\text{quantité de silicate dans le pied de cuve, exprimée en SiO}_2}$$

inférieur ou égal à 7, de préférence allant de 4 à 6,5,
- b) préneutralisation dudit pied de cuve par l'agent acidifiant jusqu'à neutralisation de 50 à 85% de la quantité de M$_2$O présente ;
- c) introduction dans ledit pied de cuve préneutralisé de la fraction restante de silicate de métal alcalin en solution aqueuse et de l'agent acidifiant, dans des conditions telles que le pH du milieu réactionnel reste sensiblement constant et de 8,6 à 9,6 ;
- d) arrêt de l'addition de silicate
- e) poursuite de l'acidification du milieu réactionnel jusqu'à obtenir une bouillie de silice de pH de 3,7 à 4,6 ;

ledit procédé étant caractérisé en ce que, après arrêt de l'addition de silicate à l'étape d) et préalablement à l'étape e), le milieu est amené à un pH de 7 à 8 par addition d'agent acidifiant puis soumis à un mûrissement pendant 5 à 30 minutes. La silice obtenue selon le procédé de l'invention, présente une surface spécifique CTAB de 20 à 80m$^2$/g, de préférence de 30 à 60 m$^2$/g.

La surface spéctitque BET est déterminée selon la méthode de BRUNAUER - EMET - TELLER décrite dans "The journal of the American Chemical Society", Vol. 60, page 309, février 1938 et correspondant à la norme NFT 45007 (novembre 1987).

La surface spécifique CTAB est la surface externe déterminée selon la norme NFT 45-007 (novembre 1987) (5.12).

L'abrasivité RDA ("Radioactive Dentine Abrasion") est mesurée selon la méthode décrite par J.J. HEFFERREN dans "Journal of Dental Research", vol. 55(4), pages 563-573, 1976.

Selon cette méthode, les dents humaines irradiées par un flux de neutrons sont soumises à un certain brossage mécanique ; l'indice d'abrasion de la silice testée correspond à la radioactivité $^{32}$P émanant de la dentine. On choisit comme référence une suspension contenant 10 grammes de pyrophosphate de calcium dispersé dans 50ml d'une solution eau/glycérine 90/10 en volume et contenant 0,5% de carboxyméthylcellulose de sodium, référence dont la RDA est fixée arbitrairement à 100. La silice dont on veut mesurer la RDA est mise en suspension comme le pyrophosphate de calcium et soumise au même brossage mécanique.

La prise d'huile DOP est déterminée selon la norme ISO 787/5 en mettant en oeuvre du dioctylphtalate.

L'indice de réfraction mesuré dans le sorbitol est celui de la suspension la plus transparente (donc transmission maximum) de cette silice dans diverses solutions eau-sorbitol, transparence déterminée par la transmission à 589 nm avec un spectrophotomètre. Chaque suspension est obtenue par dispersion d'un gramme de silice dans 19 grammes de solution eau-sorbitol, puis désaération sous léger vide avant lecture de la tranmission (lecture faite avec, comme produit de référence, la solution eau-sorbitol sans silice) sur le spectrophotomètre et de l'indice de réfraction sur un réfractomètre.

La diamètre médian en poids $d_{50}$ des particules de silice est déterminé à l'aide d'un appareil SYMPATEC HELOS. Cet appareil applique le principe de la diffraction FRAUNHOFFER et met en oeuvre un laser He/Ne de faible puissance. L'échantillon est préalablement dispersé par application d'ultra-sons dans l'eau pendant 30 secondes pour obtenir une suspension aqueuse.

Le choix du silicate et de l'agent acidifiant pour réaliser le procédé de l'invention, se fait d'une manière bien connue en soi.

Le silicate de métal alcalin est avantageusement un silicate de sodium ou de potassium. On peut citer tout particulièrement les silicates de sodium.

Ledit silicate est mis en oeuvre sous forme d'une solution aqueuse présentant une concentration exprimée en $SiO_2$, de 50 à 350 g/l, de préférence de 100 à 250 g/l.

On utilise généralement comme agent acidifiant un acide minéral fort tel que l'acide sulfurique, l'acide nitrique, l'acide chlorhydrique ou un acide organique comme l'acide acétique, l'acide formique ou l'acide carbonique. D'une manière préférentielle, il s'agit de l'acide sutfurique. Ce dernier peut être mis en oeuvre sous forme dilué ou concentré, de préférence sous forme d'une solution aqueuse présentant une concentration de 40 à 400g/l, de préférence de 60 à 400g/l.

Parmi les electrolytes on peut citer notamment les sels de métaux alcalins ou alcalino-terreux, notamment le sel du métal du silicate de départ et de l'agent acidifiant, à savoir de préférence le sulfate de sodium ; les chlorure, nitrate et hydrogénocarbonate de sodium sont également intéressants.

La première étape a) consiste à former le pied de cuve constitué d'eau, d'au moins un sel électrolyte et d'une fraction de la quantité totale de silicate.

La concentration en silicate dans le pied de cuve Initial est de 10 à 100 g, de préférence de 25 à 90 g de $SiO_2$ par litre de pied de cuve.

La quantité de sel électrolyte présente dans le pied de cuve peut être de 0,05 à 0,3 mole/litre lorsqu'il s'agit d'un sel électrolyte de métal alcalin ou de 0,005 à 0,05 mole/litre lorsqu'il s'agit d'un sel électrolyte de métal alcalino-terreux.

Le pied de cuve obtenu est porté à une température de 70 à 98°C, de préférence de 80 à 95°C, et maintenu sous agitation.

La deuxième étape b) consiste à ajouter, dans les mêmes conditions, de l'agent acidifiant audit pied de cuve initial, jusqu'à ce que de 50 à 85%, de préférence de 55 à 80% de la quantité de $M_2O$ présente soient neutralisés.

La troisième étape c) consiste à ajouter au pied de cuve préneutralisé maintenu sous agitation, la fraction restante de solution de silicate et l'agent acidifiant simultanément.

Les quantités respectives de silicate de métal alcalin et d'agent acidifiant sont choisies de manière à maintenir le pH du milieu réactionnel à une valeur sensiblement constante de 8,6 à 9,6, de préférence de 9 à 9,4, pendant toute l'introduction des deux réactifs.

Ces deux solutions sont Introduites en maintenant le milieu à une température de 70 à 98°C, de préférence de 80 à 95°C.

On arrête ensuite l'introduction de la solution de silicate (étape d)) afin d'obtenir un taux de consolidation TC, tel que défini ci-dessus, inférieur ou égal à 7, de préférence de 4 à 6,5.

La troisième étape c) dure généralement de 20 à 50 minutes.

L'addition d'agent acidifiant dans le milieu réactionnel sous agitation est ensuite poursuivie , dans les mêmes conditions de température, jusqu'à obtention d'un pH du milieu de 7 à 8, de préférence de 7,3 à 7,8.

Le milieu est ensuite laissé mûrir dans les mêmes conditions de température, pendant 5 à 30 minutes de préférence pendant 5 à 10 minutes, avant réintroduction de l'agent acidifiant pour réaliser la cinquième étape e) jusqu'à obtention d'un pH du milieu (bouillie acidifiée) de 3,7 à 4,6, de préférence de 3,9 à 4,5.

En fin de cinquième étape, après arrêt total de l'addition d'agent acidifiant, on laisse mûrir le milieu réactionnel dans les mêmes conditions de température. Cette opération de mûrissement peut durer de 5 à 30 minutes, de préférence de 5 à 10 minutes.

On obtient à l'issue des opérations ci-dessus décrites une bouillie de silice, qui est ensuite séparée (séparation liquide-solide) ; cette opération consiste généralement en une filtration (par exemple à l'aide d'un filtre rotatif sous vide), suivie d'un lavage à l'eau.

On récupère ainsi une suspension de silice (gâteau de filtration), qui est ensuite séchée, de préférence par atomisation (par exemple atomiseur à turbines).

La silice ainsi obtenue est broyée, si nécessaire, jusqu'à obtention d'un diamètre médian $d_{50}$ de particules de 6 à 13µm, de préférence de 7 à 12µm.

La silice obtenue selon le procédé ci-dessus décrit, présente l'avantage par incorporation dans une composition dentifrice, de conférer à ladite composition dentifrice un pouvoir abrasif faible ou moyen sans risque de dommages pour les tissus oraux, tout en apportant un pouvoir nettoyant suffisant pour éliminer efficacement la plaque dentaire et les débris divers. Cette silice est donc tout particulièrement adaptée à la formulation de dentifrices pour usage fréquent.

Le pouvoir abrasif RDA des pâtes dentifrices est également déterminé selon la méthode décrite par J.J. HEFFERREN dans "Journal of Dental Research", vol. 55(4), pages 563-573, 1976, à l'aide de 25g de pâte dentifrice.

Le pouvoir nettoyant des dentifrices est déterminé selon la méthode décrite par G.K. STOOKEY, T.A. BURKHARD et B.R. SCHEMEHORN dans "Journal of Dental Research", Vol. 61, n° 11, novembre 1982, pages 1236-1239.

La silice obtenue selon le procédé de l'invention peut être présente dans les compositions dentifrices à raison de 5 à 50%, de préférence de 5 à 30% du poids desdites compositions.

Les compositions dentifrices comprenant la silice obtenue selon le procédé faisant l'objet de l'invention présentent généralement une abrasivité RDA, mesurée comme ci-dessus indiqué, inférieure à 55 pour une quantité de silice de l'invention de 10g pour 100g de pâte, avec un rapport pouvoir nettoyant / RDA supérieur à 1,5.

Ces compositions peuvent en outre renfermer d'autres ingrédients habituels, en particulier des agents abrasifs minéraux autres, insolubles dans l'eau, des agents épaississants, des humectants ...

Comme agents abrasifs autres, on peut mentionner en particulier le carbonate de calcium, l'alumine hydratée, la bentonite, le silicate d'aluminium, le silicate de zirconium, les métaphosphates et phosphates de sodium, de potassium, de calcium et de magnésium. La quantité totale de poudre(s) abrasive(s) peut constituer de l'ordre de 5 à 50% du poids de la composition dentaire.

Parmi les agents épaississants, on peut mentionner tout particulièrement les silices épaississantes en quantité de l'ordre de 1 à 15 % du poids, la gomme xanthane, la gomme guar, les carraghénanes, les dérivés de la cellulose, les alginates, en quantité pouvant aller jusqu'à 5% du poids de ladite composition ....

Parmi les agents humectants, on peut citer par exemple le glycérol, le sorbitol, les polyethylène glycols, les polypropylène glycols, le xylitol, en quantité de l'ordre de 2 à 85%, de préférence de l'ordre de 10 à 70% du poids de composition dentifrice exprimé en sec.

Ces compositions dentifrices peuvent en outre comporter des agents tensio-actifs, des agents détergents, des colorants, des anti-bactériens, des dérivés fluorés, des opacifiants, des arômes, des édulcorants, des agents antitartre, antiplaque, des agents de blanchiment, du bicarbonate de sodium, des antiseptiques, des enzymes, des extraits naturels (camomille, thym ...) ...

Les exemples suivants sont donnés à titre illustratif.


**Exemple 1**

Préparation du pied de cuve

On introduit dans un réacteur de 25 litres,

.   2,2 litres d'eau de ville
.   966g d'une solution aqueuse de silicate de sodium de rapport $SiO_2/ Na_2O$ de 3,5 contenant 135g/l de $SiO_2$
.   72g de sulfate de sodium.

La température est amenée à 90°C sous forte agitation.

Préneutralisation

On ajoute ensuite, dans les mêmes conditions, une solution d'acide sulfurique à 80g/l, jusqu'à neutralisation de 71% du pied de cuve.

Addition simultanée

On introduit ensuite simultanément une solution aqueuse de silicate de sodium de rapport $SiO_2/ Na_2O$ de 3,5 contenant 135g/l de $SiO_2$ et une solution aqueuse à 80g/l d'acide sulfurique, pendant 30 minutes, de manière à atteindre un pH sensiblement constant de 9-9,2, et ce jusqu'à l'obtention d'un taux de consolidation TC de 6,2.

1ère acidification

Après arrêt de l'introduction de silicate, on prolonge l'addition de solution d'acide sulfurique dans les mêmes conditions, jusqu'à obtention d'un pH de 7,5.

Mûrissement

On arrête l'addition d'acide et on laisse mûrir le milieu réactionnel pendant 5 minutes dans les mêmes conditions de température.

2ème acidification

On introduit à nouveau la solution d'acide sulfurique, dans les mêmes conditions, jusqu'à obtention d'un pH de 4,2.
Le produit est ensuite filtré, lavé et séché par atomisation.
Les caractéristiques du produit obtenu sont données au tableau 1.

**Exemple 2**

Préparation du pied de cuve

On introduit dans un réacteur de 2m$^3$,

- 94 litres d'eau de ville
- 125 litres d'une solution aqueuse de silicate de sodium de rapport $SiO_2/ Na_2O$ de 3,5 contenant 135g/l de $SiO_2$
- 4kg de sulfate de sodium,

ce qui correspond à 77g/litre de $SiO_2$ et 18,3g/litre de sulfate de sodium dans le pied de cuve.
La température est amenée à 92°C sous forte agitation.

Préneutralisation

On ajoute ensuite, dans les mêmes conditions, une solution d'acide sulfurique à 80g/l, jusqu'à neutralisation de 71% du pied de cuve.

Addition simultanée

On introduit ensuite simultanément une solution aqueuse de silicate de sodium de rapport $SiO_2/ Na_2O$ de 3,5 contenant 135g/l de $SiO_2$ et une solution aqueuse à 80g/l d'acide sulfurique, pendant 40 minutes, de manière à atteindre un pH sensiblement constant de 9-9,2 et ce jusqu'à l'obtention d'un taux de consolidation TC de 7.

1ère acidification

Après arrêt de l'introduction de silicate, on prolonge l'addition de solution d'acide sulfurique, dans les mêmes conditions, jusqu'à obtention d'un pH de 7,5.

Mûrissement

On arrête l'addition d'acide et on laisse mûrir le milieu réactionnel pendant 5 minutes dans les mêmes conditions de température.

2ème acidification

On introduit à nouveau la solution d'acide sulfurique, dans les mêmes conditions jusqu'à obtention d'un pH de 4,2.
Le produit est ensuite filtré, lavé, séché par atomisation et broyé.

Les caractéristiques du produit obtenu sont données au tableau 1.

**Exemples 3-5**

On répète les opérations décrites à l'exemple 2 en y apportant les modifications figurant au tableau 1. Les caractéristiques des produits obtenus sont données au tableau 1.

**Exemples 6 et 7 comparatifs**

On répète les opérations décrites à l'exemple 2 en y apportant les modifications figurant au tableau 1. Les caractéristiques des produits obtenus sont données au tableau 1.
Les exemples 1 à 5 ci-dessus réalisés avec un taux de consolidation TC allant jusqu'à 7, montrent que l'abrasivité RDA des silices obtenues est d'au plus 75, tandis que la mise en oeuvre d'un taux de consolidation TC de 8 (exemples comparatifs 6 et 7) conduit à des silices d'abrasivité RDA d'au moins 97.

**Exemples 8-10**

Les silices des exemples 2, 4 et 7 comparatif, sont mises en oeuvre comme agent abrasif dans la préparation de pâte dentifrice, dont la composition est donnée au tableau 2.
Le pouvoir abrasif RDA et le pouvoir nettoyant de ces pâtes figurent au tableau 2.
On constate que le pouvoir nettoyant des pâtes contenant 10% de leur poids de silice de l'invention (silice des exemples 2 et 4) est au moins aussi élevé que celui des pâtes contenant la même quantité de silice standard ; le rapport pouvoir nettoyant / RDA des pâtes selon l'invention est donc particulièrement élevé.

Tableau 1

| exemple | 1 | 2 | 3 | 4 | 5 | 6 comparatif | 7 comparatif |
|---|---|---|---|---|---|---|---|
| **procédé** | | | | | | | |
| pied de cuve | | | | | | | |
| eau (litre) | 2,2 | 94 | 101 | 101 | 101 | 78 | 78 |
| silicate (litre) | 0,85 | 125 | 148 | 135 | 135 | 104 | 104 |
| sulfate de sodium (kg) | 0,072 | 4 | 5,6 | 5,6 | 5,6 | 1,1 | 4,3 |
| $SiO_2$ (g/l) | 38 | 77 | 80 | 77 | 77 | 77 | 77 |
| Sulfate de sodium (g/l) | 24 | 18,3 | 22,5 | 23,7 | 23,7 | 6 | 23,6 |
| préneutralisation | | | | | | | |
| taux (%) | 71 | 71 | 65 | 72 | 57 | 71 | 71 |
| addition simultanée | | | | | | | |
| TC | 6,2 | 7 | 5,6 | 6 | 5,25 | 8 | 8 |
| durée (minutes) | 30 | 40 | 40 | 40 | 41,5 | 40 | 40 |
| **caractéristiques** | | | | | | | |
| BET ($m^2$/g) | 109 | 112 | 89 | 73 | 83 | 91 | 70 |
| CTAB ($m^2$/g) | 32 | 43 | 42 | 44 | 48 | 36 | 35 |
| DOP ml/100g) | 146 | 122 | 140 | 137 | 151 | 120 | 96 |
| RDA | 68 | 75 | 62 | 66 | 51 | 97 | 125 |
| IR | 1,445 | 1,445 | 1,445 | 1,445 | 1,446 | 1,444 | 1,442 |
| $d_{50}$ (µm) | 8,3 | 9,1 | 9,1 | 8,5 | 8,3 | 9,3 | 9,1 |

Tableau 2

| formulation | | | |
|---|---|---|---|
| sorbitol (solution à 70%) | 49% | 49% | 49% |

Tableau 2   (suite)

| formulation | | | |
|---|---|---|---|
| silice de l'exemples 2 | 10% | | |
| silice de l'exemple 4 | | 10% | |
| silice de l'exemple 7 | | | 10% |
| silice épaississante TIXOSIL 43 | 9% | 9% | 9% |
| laurylsulfate de sodium | 1,3% | 1,3% | 1,3% |
| dioxyde de titane | 1% | 1% | 1% |
| benzoate de sodium | 0,2% | 0,2% | 0,2% |
| carboxyméthylcellulose de sodium | 0,9% | 0,9% | 0,9% |
| saccharinate de sodium | 0,2% | 0,2% | 0,2% |
| fluorure de sodium | 0,24% | 0,24% | 0,24% |
| arôme | 1% | 1% | 1% |
| eau désionisée (qsp 100%) | 27,16% | 27,16% | 27,16% |
| **caractéristiques** | | | |
| RDA | 42 | 38 | 70 |
| pouvoir nettoyant (%) | 75 | 63 | 73 |
| pouvoir nettoyant / RDA | 1,79 | 1,66 | 1,04 |

**Revendications**

1.  Procédé de préparation d'une silice précipitée pour composition dentifrice présentant :

    - une surface spécifique BET de 60 à 150m$^2$/g, de préférence de 70 à 130m$^2$/g ;
    - une abrasivité RDA de 30 à 90, de préférence de 40 à 80, tout particulièrement de 40 à 70 ;
    - un indice de réfraction de 1,440 à 1,450 ;
    - une prise d'huile DOP de 115 à 170 ml/100g, de préférence de plus de 120 ml/100g à 170 ml/100g ;
    - un diamètre médian de particules $d_{50}$ de 6 à 13μm, tout particulièrement de 7 à 12μm ;
    - et, lorsqu'elle est formulée à 10% en poids dans une composition dentifrice, un rapport pouvoir nettoyant / abrasivité RDA dans la composition dentifrice supérieur à 1,5 ;

    par réaction d'un silicate de métal alcalin M , de rapport SiO$_2$ / M$_2$O de 2 à 4, de préférence de 3 à 3,8 , avec un agent acidifiant, éventuellement mûrissement de la bouillie de silice formée, séparation et séchage de la suspension de silice récupérée et broyage si nécessaire jusqu'à obtention d'un diamètre médian $d_{50}$ de particules de 6 à 13μm, de préférence de 7 à 12μm, l'opération de formation de la bouillie de silice étant réalisée selon les étapes successives suivantes :

    - a) mise en oeuvre d'un pied de cuve Initial constitué d'eau, d'un sel électrolyte et d'une fraction de la quantité totale de silicate engagée correspondant à un taux de consolidation TC

$$TC = \frac{\text{quantité totale de silicate engagée, exprimée en SiO2}}{\text{quantité de silicate dans le pied de cuve, exprimée en SiO}_2}$$

    inférieur ou égal à 7, de préférence allant de 4 à 6,5,
    - b) préneutralisation dudit pied de cuve par l'agent acidifiant jusqu'à neutralisation de 50 à 85% de la quantité de M$_2$O présente ;
    - c) introduction dans ledit pied de cuve préneutralisé de la fraction restante de silicate de métal alcalin en solution aqueuse et de l'agent acidifiant, dans des conditions telles que le pH du milieu réactionnel reste sensiblement constant et de 8,6 à 9,6 ;
    - d) arrêt de l'addition de silicate
    - e) poursuite de l'acidification du milieu réactionnel jusqu'à obtenir une bouillie de silice de pH de 3,7 à 4,6 ;

    ledit procédé étant **caractérisé en ce que**, après arrêt de l'addition de silicate à l'étape d) et préalablement à l'étape e), le milieu est amené à un pH de 7 à 8 par addition d'agent acidifiant puis soumis à un mûrissement pendant 5 à 30 minutes.

**2.** Procédé selon la revendication 1), **caractérisé en ce que** le silicate de métal alcalin est un silicate de sodium.

**3.** Procédé selon la revendication 1) ou 2), **caractérisé en ce que** le silicate est mis en oeuvre sous forme d'une solution aqueuse présentant une concentration exprimée en $SiO_2$, de 50 à 350 g/l, de préférence de 100 à 250 g/l.

**4.** Procédé selon l'une quelconque des revendications 1) à 3), **caractérisé en ce que** la concentration en silicate dans le pied de cuve initial est de 10 à 100, de préférence de 25 à 90 g de $SiO_2$ par litre de pied de cuve.

**5.** Procédé selon l'une quelconque des revendications 1) à 4), **caractérisé en ce que** l'électrolyte est un sel de métal alcalin ou alcalino-terreux.

**6.** Procédé selon la revendication 5), **caractérisé en ce que** l'électrolyte est du sulfate, du chlorure, du nitrate ou de l'hydrogénocarbonate de sodium.

**7.** Procédé selon l'une quelconque des revendications 1) à 6), **caractérisé en ce que** la quantité de sel électrolyte présente dans le pied de cuve est de 0,05 à 0,3 mole/litre lorsqu'il s'agit d'un sel électrolyte de métal alcalin ou de 0,005 à 0,05 mole/litre lorsqu'il s'agit d'un sel électrolyte de métal alcalino-terreux.

**8.** Procédé selon l'une quelconque des revendications 1) à 7), **caractérisé en ce que** l'agent acidifiant est de l'acide sulfurique, de l'acide nitrique, de l'acide chlorhydrique, de l'acide acétique, de l'acide formique ou de l'acide carbonique.

**9.** Procédé selon la revendication 8), **caractérisé en ce que** l'agent acidifiant est de l'acide sulfurique.

**10.** Procédé selon la revendication 9), **caractérisé en ce que** l'acide sulfurique est mis en oeuvre sous forme d'une solution aqueuse présentant une concentration de 40 à 400g/l, de préférence de 60 à 400g/l.

**11.** Procédé selon l'une quelconque des revendications 1) à 10), **caractérisé en ce que** les différentes étapes de réalisation de formation de la bouillie de silice sont effectuées à une température de 70 à 98°C, de préférence de 80 à 95°C.

## Claims

**1.** Process for preparing a precipitated silica for a toothpaste composition having:

- a BET specific surface from 60 to 150 $m^2/g$, preferably from 70 to 130 $m^2/g$;
- an RDA abrasiveness from 30 to 90, preferably from 40 to 80, most particularly from 40 to 70;
- a refractive index from 1.440 to 1.450;
- a DOP oil uptake from 115 to 170 ml/100 g, preferably from more than 120 ml/100 g to 170 ml/100 g;
- an average particle diameter $d_{50}$ from 6 to 13 $\mu m$, most particularly from 7 to 12 $\mu m$; and
- and, when it is formulated at 10% by weight in a toothpaste composition, a cleaning power/RDA abrasiveness ratio in the toothpaste composition of greater than 1.5;

by reaction of an alkali metal M silicate, with an $SiO_2/M_2O$ ratio from 2 to 4, preferably from 3 to 3.8, with an acidifying agent, optional maturation of the silica broth formed, separation and drying of the silica suspension recovered and grinding if necessary, until an average particle diameter $d_{50}$ from 6 to 13 $\mu m$, preferably from 7 to 12 $\mu m$, is obtained, the operation for formation of the silica broth being carried out according to the following successive steps:

-a) using an initial feedstock consisting of water, an electrolytic salt and a fraction of the total amount of silicate employed corresponding to a consolidation ratio CR

$$CR = \frac{\text{Total amount of silicate employed, expressed as SiO}_2}{\text{Total amount of silicate in the feedstock, expressed as SiO}_2}$$

less than or equal to 7, preferably from 4 to about 6.5,

-b) preneutralizing the said feedstock with the acidifying agent until about 50 to 85% of the amount of $M_2O$ present has been neutralized;

-c) introducing into the said preneutralized feedstock the remaining fraction of alkali metal silicate in aqueous solution and the acidifying agent, under conditions such that the pH of the reaction medium remains substantially constant and from 8.6 to 9.6;

-d) stopping the addition of silicate

-e) continuing the acidification of the reaction medium until a silica broth with a pH of 3.7 to 4.6 is obtained;

the said process being **characterized in that**, after stopping the addition of silicate in step d) and prior to step e), the medium is brought to a pH of 7 to 8 by adding acidifying agent and is then subjected to maturation for 5 to 30 minutes.

2. Process according to Claim 1, **characterized in that** the alkali metal silicate is a sodium silicate.

3. Process according to Claim 1 or 2, **characterized in that** the silicate is used in the form of an aqueous solution with a concentration, expressed as $SiO_2$, from 50 to 350 g/l, preferably from 100 to 250 g/l.

4. Process according to any one of Claims 1 to 3, **characterized in that** the silicate concentration in the initial feedstock is from 10 to 100, preferably from 25 to 90 g of $SiO_2$ per litre of feedstock.

5. Process according to any one of Claims 1 to 4, **characterized in that** the electrolyte is an alkali metal or alkaline-earth metal salt.

6. Process according to Claim 5, **characterized in that** the electrolyte is sodium sulphate, chloride, nitrate or hydrogen carbonate.

7. Process according to any one of Claims 1 to 6, **characterized in that** the amount of electrolytic salt present in the feedstock is from 0.05 to 0.3 mol/litre when it is an electrolytic salt of an alkali metal or from 0.005 to 0.05 mol/litre when it is an electrolytic salt of an alkaline-earth metal.

8. Process according to any one of Claims 1 to 7, **characterized in that** the acidifying agent is sulphuric acid, nitric acid, hydrochloric acid, acetic acid, formic acid or carbonic acid.

9. Process according to Claim 8, **characterized in that** the acidifying agent is sulphuric acid.

10. Process according to Claim 9, **characterized in that** the sulphuric acid is used in the form of an aqueous solution with a concentration from 40 to 400 g/l, preferably from 60 to 400 g/l.

11. Process according to any one of Claims 1 to 10, **characterized in that** the various steps in the formation of the silica broth are carried out at a temperature from 70 to 98°C, preferably from 80 to 95°C.

**Patentansprüche**

1. Verfahren zur Herstellung einer gefällten Kieselsäure für eine Zahnpflegemittelzusammensetzung, die

- eine spezifische BET-Oberfläche von 60 bis 150 m$^2$/g, vorzugsweise 70 bis 130 m$^2$/g;

- eine RDA-Abrasivität (Abriebvermögen) von 30 bis 90, vorzugsweise von 40 bis 80, insbesondere von 40 bis 70;

- einen Brechungsindex von 1,440 bis 1,450;

- eine Ölzahl DOP von 115 bis 170 ml/100 g, vorzugsweise von mehr als 120 ml/100 g bis 170 ml/100 g;

- einen mittleren Durchmesser der Teilchen $d_{50}$ von 6 bis 13 μm, insbesondere von 7 bis 12 μm;

- und, wenn sie zu 10 Gew.-% in einer Zahnmittelzusammensetzung formuliert ist, ein Verhältnis von Reinigungsvermögen/RDA-Abrasivität in der Zahnpflegemittelzusammensetzung von mehr als 1,5 aufweist;

durch Reaktion eines Silikats eines Alkalimetalls M mit einem $SiO_2/M_2O$-Verhältnis von 2 bis 4, vorzugsweise von 3 bis 3,8, mit einem sauermachenden Mittel, gegebenenfalls Reifenlassen des gebildeten Kieselsäureschlickers, Trennung und Trocknung der erhaltenen Kieselsäuresuspension und, falls erforderlich, Zerkleinern bis zum Erhalt eines mittleren Durchmessers $d_{50}$ der Teilchen von 6 bis 13 μm, vorzugsweise 7 bis 12 μm, wobei der Verfahrensschritt der Bildung des Kieselsäureschlickers entsprechend den folgenden aufeinanderfolgenden Schritten durchgeführt wird:

a) Ansetzen einer anfänglichen Vorlage, bestehend aus Wasser, einem Elektrolyten und einem Anteil der Gesamtmenge des eingesetzten Silikats entsprechend einem Verfestigungsgrad TC

$$TC = \frac{\text{Gesamtmenge des eingesetzten Silikats, berechnet als } SiO_2}{\text{Menge des Silikats in der Vorlage, berechnet als } SiO_2}$$

von kleiner oder gleich 7, vorzugsweise von 4 bis 6,5,

b) Vorneutralisierung der Vorlage durch ein sauermachendes Mittel bis zur Neutralisierung von 50 bis 85 % der vorhandenen $M_2O$-Menge,

c) Zugabe des restlichen Anteils des Alkalimetallsilikats in wäßriger Lösung und des sauermachenden Mittels zu der vorneutralisierten Vorlage unter solchen Bedingungen, daß der pH-Wert des Reaktionsmilieus im wesentlichen konstantbleibt und 8,6 bis 9,6 beträgt,

d) Stop der Zugabe des Silikats,

e) weiteres Ansäuern des Reaktionsmilieus bis zum Erhalt eines Kieselsäureschlickers mit einem pH-Wert von 3,7 bis 4,6,

**dadurch gekennzeichnet, daß** nach dem Stop der Zugabe des Silikats in Verfahrensschritt d) und vor Verfahrensschritt e) das Milieu durch Zugabe eines sauermachenden Mittels auf einen pH-Wert von 7 bis 8 gebracht und dann einer Reifung für 5 bis 30 Minuten unterworfen wird.

2. Verfahren nach Anspruch 1, **dadurch gekennzeichnet, daß** das Alkalimetallsilikat ein Natriumsilikat ist.

3. Verfahren nach Anspruch 1 oder 2, **dadurch gekennzeichnet, daß** das Silikat in Form einer wäßrigen Lösung mit einer Konzentration, berechnet als $SiO_2$, von 50 bis 350 g/l, vorzugsweise 100 bis 250 g/l, eingesetzt wird.

4. Verfahren nach einem der Ansprüche 1 bis 3, **dadurch gekennzeichnet, daß** die Konzentration an Silikat in der anfänglichen Vorlage 10 bis 100 g $SiO_2$ pro Liter Vorlage, vorzugsweise 25 bis 90 g $SiO_2$ pro Liter Vorlage, beträgt.

5. Verfahren nach einem der Ansprüche 1 bis 4, **dadurch gekennzeichnet, daß** der Elektrolyt ein Alkalimetall- oder Erdalkalimetallsalz ist.

6. Verfahren nach Anspruch 5, **dadurch gekennzeichnet, daß** der Elektrolyt Natriumsulfat, -chlorid, -nitrat oder -hydrogencarbonat ist.

7. Verfahren nach einem der Ansprüche 1 bis 6, **dadurch gekennzeichnet, daß** die Menge an Elektrolytsalz in der Vorlage 0,05 bis 0,3 mol/Liter beträgt, wenn es sich um ein Alkalimetallelektrolytsalz handelt, oder 0,005 bis 0,05 mol/Liter beträgt, wenn es sich um eine Erdalkalimetallelektrolytsalz handelt.

8. Verfahren nach einem der Ansprüche 1 bis 7, **dadurch gekennzeichnet, daß** das sauermachende Mittel Schwefelsäure, Salpetersäure, Salzsäure, Essigsäure, Ameisensäure oder Kohlensäure ist.

9. Verfahren nach Anspruch 8, **dadurch gekennzeichnet, daß** das sauermachende Mittel Schwefelsäure ist.

10. Verfahren nach Anspruch 9, **dadurch gekennzeichnet, daß** die Schwefelsäure in Form einer wäßrigen Lösung mit einer Konzentration von 40 bis 400 g/l, vorzugsweise 60 bis 400 g/l, eingesetzt wird.

11. Verfahren nach einem der Ansprüche 1 bis 10, **dadurch gekennzeichnet, daß** die verschiedenen Verfahrensschritte der Bildung des Kieselsäureschlickers bei einer Temperatur von 70 bis 98 °C, vorzugsweise 80 bis 95 °C, durchgeführt werden.